# EUROPEAN PATENT APPLICATION

(11) **EP 4 538 281 A1**
(43) Date of publication of application: **16.04.2025**
(21) Application number: 23820005.9
(22) Date of filing: 12.05.2023
(51) Int. Cl.: C07K 7/06, A61K 38/00, A61P 21/00, A23L 33/18

(54) **PEPTIDE EXHIBITING MUSCLE LOSS PREVENTION AND MUSCLE MASS PROMOTION ACTIVITIES, AND USE THEREOF**

(30) Priority: 10.06.2022 KR 20220071033
(71) Applicant: Caregen Co., Ltd., Anyang-si, Gyeonggi-do 14119 (KR)
(72) Inventor: CHUNG, Yong Ji, Anyang-si, Gyeonggi-do 14119 (KR); KIM, Eun Mi, Anyang-si, Gyeonggi-do 14119 (KR); KIM, Seon Soo, Anyang-si, Gyeonggi-do 14119 (KR)
(74) Representative: Potter Clarkson
(86) International application number: PCT/KR2023/006480
(87) International publication number: WO 2023/239069

(57) **Abstract**

The present application relates to a peptide exhibiting muscle loss prevention and muscle mass promotion activities, and use thereof, and provides: a peptide consisting of an amino acid sequence of SEQ ID NO: 1; a pharmaceutical composition for preventing or treating a muscle disease, comprising the peptide as an active ingredient; and a food composition for preventing or ameliorating a muscle disease, comprising the peptide as an active ingredient.

## Description

### Technical Field

The present application relates to a peptide exhibiting muscle loss prevention and muscle mass promotion activities, and use thereof. This application is based on and claims priority to Korean Patent Application No. 10-2022-0071033, filed on June 10, 2022, in the Korean Intellectual Property Office, the disclosure of which is incorporated by reference herein in its entirety.

### Background Art

The aging population has been growing rapidly worldwide in recent years, with the United Nations predicting that by 2050, there will be more than 2 billion people aged 60 or older. Skeletal muscle is the largest organ in the body, accounting for 50 % of total body weight, and muscle loss and weakness is one of the major body changes that occur with aging. In this regard, skeletal muscle tends to decline in the body at a rate of about 1 % per year after the age of 30, and then declines rapidly after the age of 65. This rapid loss of muscle can have a significant impact on quality of life, reducing physical performance and increasing the risk of falls and fractures, and in response to these concerns, the World Health Organization (WHO) officially recognized sarcopenia as a disease in 2017.

Sarcopenia, which is caused by degeneration of spinal nerves, motor nerves, or skeletal muscle fibers associated with muscle disease, is one of the representative intractable diseases with no known cause. According to research to date, it is known that the motor nerves that induce contraction of skeletal muscles degenerate such that the contraction of skeletal muscles does not proceed, or the expression of proteins involved in muscle contraction within skeletal muscles is reduced or proteins are modified, such that normal contraction of skeletal muscles does not proceed, and in the long term, the motor nerves or skeletal muscles are modified into fibrous tissues. Since the underlying pathogenesis of sarcopenia has not yet been identified and no method has been developed to prevent or restore the degeneration of the motor nerves or skeletal muscles, research is currently underway to develop methods to slow the progression of sarcopenia. Currently, exercise, proteins, and calorie supplementation are known to be helpful in treating sarcopenia, but are not very helpful in the elderly who account for the majority of sarcopenia patients, and therefore a therapeutic agent for sarcopenia is urgently needed.

Meanwhile, muscle size or muscle mass is regulated by intracellular signaling processes that induce anabolic or catabolic reactions within the muscle. Specifically, when signaling responses that induce the synthesis of muscle proteins predominate over the breakdown of muscle proteins, the synthesis of muscle proteins is increased, resulting in an increase in muscle size (hypertrophy) or an increase in the number of muscle fibers (hyperplasia). Also, muscle cell differentiation and muscle formation are regulated by a variety of muscle regulatory factors. MyoD initiates the expression of muscle-specific genes and induces the differentiation of muscle satellite cells into myoblasts. The induction of myogenin expression by MyoD activity is the most important factor in the fusion of myoblasts, which is involved in the formation of myotubes. Muscle fibers formed through this process form bundles and ultimately form muscles.

Against this technical background, diverse studies are underway to inhibit muscle loss caused by external or endogenous factors and to promote the synthesis of muscle proteins (KR 10-2064387), but are still incomplete.

### Disclosure of Invention

### Technical Problem

An aspect is to provide a peptide consisting of an amino acid sequence of SEQ ID NO: 1.

Another aspect is to provide a composition for muscle loss prevention and muscle mass promotion, the composition including a peptide consisting of an amino acid sequence of SEQ ID NO: 1 as an active ingredient.

Other purposes and advantages of the present application will become more obvious with the following detailed description, claims, and drawing. Contents not described herein will be sufficiently recognized and inferred by those skilled in the technical field of the present application or in a similar technical field therewith, and thus descriptions of such contents will be omitted.

### SOLUTION TO PROBLEM

Descriptions and embodiments disclosed herein may also be applied to other descriptions and embodiments, respectively. That is, all combinations of various elements disclosed herein belong to the scope of the present application. In addition, the scope of the present application is not construed to be limited by the detailed description provided below.

An aspect provides a peptide consisting of an amino acid sequence of SEQ ID NO: 1.

The term "peptide" as used herein refers to a linear molecule in which amino acid residues bind to each other via peptide linkages. The peptide may be prepared according to chemical synthesis methods known in the art, particularly solid phase synthesis techniques or liquid phase synthesis techniques (see US5516891). The inventors of the present disclosure put a lot of effort to develop a peptide having a biologically effective activity, and consequently established a peptide consisting of an amino acid sequence of SEQ ID NO: 1. Here, the biologically effective activity may include at least one selected from the following properties: (a) enhanced expression of myogenin (MyoG) and myogenic factor 5 (Myf5), which are early differentiation markers of myoblasts; (b) enhanced expression of myosin heavy chain (MyHC), which is a late differentiation marker of myoblasts or a protein constituting muscle fibers, and α-actinin; and (c) decreased expression of α-smooth muscle actin (α-SMA), which is an inflammatory protein, and interleukin-6 (IL-6). Accordingly, the peptide may be utilized for the purpose of preventing, improving or treating a muscle disease.

The peptide may include a protecting group bound to an N-terminus or a C-terminus of the peptide to obtain chemical stability, enhanced pharmacological properties (e.g., half-life, absorbency, titer, efficacy, etc.), modified specificity (e.g., broad spectrum of biological activity), and reduced antigenicity. In an embodiment, the N-terminus of the peptide may be bound to any one protecting group selected from the group consisting of an acetyl group, a fluoreonylmethoxycarbonyl group, a formyl group, a palmitoyl group, a myristyl group, a stearyl group, a butoxycarbonyl group, an allyloxycarbonyl group, and polyethylene glycol (PEG); and/or the C-terminus of the peptide may be bound to any one protecting group selected from the group consisting of an amino group (-NH₂), a tertiary alkyl group, and an azide group(-NHNH₂). In addition, the peptide may optionally further include a targeting sequence, a tag, a labeled residue, or an amino acid sequence specifically prepared to increase half-life or peptide stability.

The peptide may be artificially synthesized, or non-naturally occurring or engineered. The "non-naturally occurring or engineered" state refers to a state created by artificial modification rather than a state occurring its natural state. Here, the artificial modification may include artificial synthesis of an amino acid sequence by mimicking a plurality of amino acid structures, or manipulation to obtain chemical stability, enhanced pharmacological properties, altered specificity, or reduced antigenicity as described above.

The term "stability" as used herein refers to storage stability (e.g., storage stability at room temperature) as well as *in vivo* stability for protecting the peptide from attacks of *in vivo* proteolytic enzymes.

Another aspect provides a pharmaceutical composition for preventing or treating a muscle disease, including the peptide consisting of an amino acid sequence of SEQ ID NO: 1 as an active ingredient.

The terms or elements mentioned in the description of the peptide are the same as those already described above.

The term "prevention" as used herein refers to any action that can inhibit or delay the onset of a disease by administration of the composition.

The term "treatment" as used herein refers to any form of treatment that provides, to a subject afflicted with or at risk of developing a disease, effects including improving conditions (e.g., one or more symptoms) of the subject, delaying progression of a disease, delaying onset of symptoms, or slowing progression of symptoms. That is, the terms "treatment" and "prevention" are not intended to mean cure or complete elimination of symptoms.

The term "muscle disease" as used herein refers to a muscle disease, disorder, or condition associated with decrease in proliferation or differentiation of myoblasts, and for example, may collectively refer to a muscle disease resulting from degraded muscle functions, muscle loss, or muscle degeneration. The muscle disease may be, for example, sarcopenia, atony, muscular dystrophy, muscular atrophy, muscle degeneration, myotonia, amyotrophic lateral sclerosis, myasthenia, and cachexia, but is not limited thereto.

In an embodiment, the pharmaceutical composition according to an embodiment may increase muscle mass or muscle strength or improve muscle functions, by promoting proliferation and differentiation of myoblasts.

Meanwhile, functional peptides in the art, despite effective biological activity thereof, were not effectively introduced to into target tissues or cells due to the size of the peptides themselves, or had a short half-life so that they disappeared from the body in a short period of time. On the other hand, the pharmaceutical composition according to an embodiment includes, as an active ingredient, a peptide consisting of about 10 or fewer amino acids, resulting in excellent cell penetration rates of the active ingredient. For example, when administered topically, an effective therapeutic effect of a muscle disease may be obtained.

According to an embodiment, the peptides disclosed herein may promote differentiation into muscle cells or muscle fibers by significantly increasing the expression of early differentiation markers (e.g., MyoG, and Myf5) and late differentiation markers of myoblasts or the expression of proteins constituting muscle fibers (e.g., myosin heavy chain, α-actinin). Also, the peptide disclosed herein may improve recovery/regeneration of muscle mass when muscle is damaged by exogenous factors, and may also alleviate symptoms of a muscle disease and slow down progression of the disease by reducing infiltration of immune cells and accumulation of collagen tissue in damaged muscle tissue. Accordingly, the peptide may be utilized as the active ingredient of the pharmaceutical composition for treating a muscle disease.

The pharmaceutical composition may include: a pharmaceutically effective amount of the peptide; and/or a pharmaceutically acceptable carrier, but embodiments are not limited thereto.

The term "pharmaceutically effective amount" as used herein may refer to an amount sufficient to achieve the therapeutic efficacy of the pharmaceutical composition for a bone disease.

A weight ratio of the peptide to the pharmaceutically acceptable carrier may be, for example, in a range of 500:1 to 1:500, and in an embodiment, in a range of 450:1 to 1:450, 400:1 to 1:400, 350:1 to 1:350, 300:1 to 1:300, 250:1 to 1:250, 200:1 to 1:200, 150:1 to 1:150, 100:1 to 1:100, 80:1 to 1:80, 60:1 to 1:60, 40:1 to 1:40, 20:1 to 1:20, 10:1 to 1:10, 8:1 to 1:8, 6:1 to 1:6, 4:1 to 1:4, or 2:1 to 1:2, but embodiments are not particularly limited thereto.

For the pharmaceutically acceptable carrier, any carrier conventionally used in the preparation of formulations may be used, and examples thereof may include lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil, and the like, but embodiments are not limited thereto. Suitable pharmaceutically acceptable carriers and agents are described in detail in Remington's Pharmaceutical Sciences (19th ed., 1995).

The pharmaceutical composition may further include, in addition to the ingredients above, a lubricant, a wetting agent, a sweetening agent, a flavoring agent, an emulsifier, a suspending agent, a preservative, and the like, but is not limited thereto.

The pharmaceutical composition may be administered orally or parenterally. When administered parenterally, the pharmaceutical composition may be administered via intramuscular injection, intravenous injection, subcutaneous injection, intraperitoneal injection, topical administration, transdermal administration, and the like, but embodiments are not limited thereto.

A dose of the pharmaceutical composition may be in a range of 0.0001 to 1,000 microgram (ug) per day, 0.001 to 1,000 ug per day, 0.01 to 1,000 ug per day, 0.1 to 1,000 ug per day, or 1.0 to 1,000 ug per day, but embodiments are not limited thereto. The pharmaceutical composition may be administered in various ways depending on factors such as a formulation method, an administration method, a patient's age, body weight, gender, and medical conditions, food, an administration time, an administration route, an excretion rate, and response sensitivity.

The pharmaceutical composition may be formulated into a unit dosage form or prepared in a multi-dose container by formulating a pharmaceutically acceptable carrier and/or excipient according to the method easily carried out by a person having ordinary skill in the art to which the present invention pertains.

The pharmaceutical composition may be in the form of a solution, suspension, or emulsion in an oil or aqueous medium, or in the form of an ointment, a cream, a gel, a transdermal absorbent, a cataplasma agent, a patch, a paste, an extract agent, a powdered agent, a granule, a tablet, or a capsule. The pharmaceutical composition may additionally include a dispersant and/or a stabilizer.

The peptide may be, for example, contained in nanosomes or nanoparticles to further solve cell penetration or stability issues. For example, the nanosomes may be prepared with a microfluidizer using lecithin as a raw material, and for example, may be contained within lecithin particles. For the method of preparing the nanosomes, any method known in the art may be used. A size of the nanosome particles may be in a range of 10 to 200 nm, for example, in a range of 10 to 180 nm, 10 to 160 nm, 10 to 140 nm, 10 to 120 nm, 10 to 100 nm, 10 to 80 nm, 10 to 60 nm, 10 to 40 nm, 10 to 20 nm, 50 to 200 nm, 50 to 180 nm, 50 to 160 nm, 50 to 140 nm, 50 to 120 nm, 50 to 100 nm, 50 to 80 nm, or 50 to 60 nm.

The pharmaceutical composition may additionally contain a component beneficial for the prevention or treatment of a muscle disease, and the component may be, for example, although not limited thereto, vitamin C, vitamin B1, vitamin B2, vitamin B6, vitamin H, vitamin PP, pro-vitamin B5, vitamin A, vitamin D, vitamin E, vitamin K1, or carotene, or any one component selected from mixtures thereof.

Another aspect provides a food composition for preventing or ameliorating a muscle disease, including a peptide consisting of an amino acid sequence of SEQ ID NO: 1 as an active ingredient.

The terms or elements mentioned in the descriptions of the peptide and the pharmaceutical composition are the same as those already described above.

The term "amelioration" as used herein refers to parameters associated with alleviation or treatment of a condition, and for example, may refer to any action that at least reduce severity of a symptom.

In an embodiment, the food composition according to an embodiment may increase muscle mass or muscle strength or improve muscle functions, by promoting proliferation and differentiation of myoblasts.

The food composition may include the peptide as is or may be used in combination with other foods or food ingredients, and may be used in any suitable manner in the art. The food composition may contain, in addition to the active ingredient, a food-acceptable supplementary food additive, and the mixed amount of the active ingredients may be suitably determined according to the purpose of use (prevention, health, or therapeutic treatment).

The food composition may additionally contain a component beneficial for the prevention or amelioration of a muscle disease, and the component may be, for example, although not limited thereto, vitamin C, vitamin B1, vitamin B2, vitamin B6, vitamin H, vitamin PP, pro-vitamin B5, vitamin A, vitamin D, vitamin E, vitamin K1, or carotene, or any one component selected from mixtures thereof.

The term "supplementary food additive" as used herein refers to a component that can be added to a food product as an additive, and is added when preparing a health functional food of each formulation in a suitable manner selected by those skilled in the art. Examples of the supplementary food additive may include various nutrients, vitamins, minerals (electrolytes), flavoring agents such as synthetic flavoring agents and natural flavoring agents, coloring agents and fillers, pectic acid and a salt thereof, alginic acid and a salt thereof, organic acids, protective colloid thickeners, pH regulators, stabilizers, preservatives, glycerin, alcohol, and carbonating agents used in carbonated beverages. However, types of the supplementary food additive are not limited thereto.

The food composition may include a health functional food. The term "health functional food" as used herein refers to a food product prepared and processed in the form of tablets, capsules, powder, granules, liquid, and pills by using raw materials or ingredients having useful functionality for the human body. Here, the term 'functionality' refers to having a useful effect on the structure and function of the human body for health purposes, such as regulation of nutrients or physiological actions. The health functional food may be prepared by using a method commonly used in the art, and during the preparation, raw materials and ingredients commonly added in the art may be added. In addition, the formulation of the health functional food may be prepared without limitation as long as it is a formulation recognized as a health functional food. The food composition may be prepared into various forms of formulations, and unlike general drugs, it has the advantage of not having side effects that may occur with long-term use of drugs since food is used as a raw material. Also, due to excellent portability, the health functional food according to an embodiment may be consumed as a supplement to enhance the therapeutic effect of a muscle disease.

In addition, there is no limitation on the type of health food products to which the composition according to an embodiment may be used/applied. The food composition including the peptide as an active ingredient may be prepared by mixing other appropriate supplementary ingredients that can be contained in a health functional food and known additives as selected by those skilled in the art. Examples of the food product that can be added may include meat, sausage, bread, chocolates, candies, snacks, confectioneries, pizza, ramen, other noodles, gum, dairy products including ice cream, various soups, beverages, teas, drinks, alcoholic beverages, and vitamin complexes. In addition, the food product may be added to extracts, teas, jellies, juice, and the like.

Another aspect provides a method of preventing or treating a muscle disease, including administering, to a subject, a composition including a peptide consisting of an amino acid sequence of SEQ ID NO: 1 as an active ingredient.

The terms or elements mentioned in the descriptions of the peptide, the pharmaceutical composition, the food composition, and the like are the same as those already described above.

The terms "administering" and "applying" as used herein are used interchangeably, and may refer to providing a given substance to a subject or a patient by any suitable method. In addition, these terms may refer to causing at least partial localization of the composition according to an embodiment to a desired site, or arrangement of the composition according to an embodiment into a subject by an administration route.

The term "subject" as used herein refers to a target in need of condition improvement, and more particularly, refers to a mammal including a human or a non-human primate, such as a mouse, a dog, a cat, a horse, and a cow.

The composition may be, for example, a pharmaceutical composition or a food composition, each including a peptide consisting of an amino acid sequence of SEQ ID NO: 1 as an active ingredient.

An amount of the peptide included as an active ingredient in the composition may be appropriately selected without limitation depending on the formulation of a food product/drug, a desired use, and the like. For example, the amount of the peptide to be added may be in a range of 0.01 to about 15 wt% based on the total food weight of the composition. In addition, a health beverage composition may be, for example, added in a proportion of 0.02 to 10 g, preferably 0.3 to 1 g, based on 100 ml of the peptide.

### Advantageous Effects of Invention

According to the peptide of an embodiment, the peptide may promote differentiation into muscle cells or muscle fibers by improving the expression of factors involved in the differentiation of myoblasts.

According to the peptide of an embodiment, the peptide may not only improve recovery/regeneration of muscle mass when muscle is damaged by an exogenous factor, but also slow down progression of the disease by reducing infiltration of immune cells and accumulation of collagen tissue in damaged muscle tissue.

Therefore, the peptide of an embodiment may be utilized as an active ingredient of a composition for preventing, ameliorating, or treating a muscle disease.

### Brief Description of Drawings

FIG. 1 shows the results of confirming, through western blotting, the increased expression levels of myogenin and Myf5, which are early differentiation markers of myoblasts, after addition of a peptide consisting of an amino acid sequence of SEQ ID NO: 1 to C2C12 cells.
FIG. 2 shows the results of quantitatively evaluating changes in the expression of early differentiation markers of myoblasts, after addition of a peptide consisting of an amino acid sequence of SEQ ID NO: 1 to C2C12 cells, wherein FIG. 2 (a) shows the results of confirming the expression levels of myogenin, and FIG. 2 (b) shows the results of confirming the expression levels of Myf5.
FIG. 3 shows the results of confirming, through immunofluorescence, the expression pattern of myogenin (MyoG), which is an early differentiation marker of myoblasts, after addition of a peptide consisting of an amino acid sequence of SEQ ID NO: 1 to C2C12 cells.
FIG. 4 shows the results of quantitatively evaluating the expression level of myogenin (Myo G), after addition of a peptide consisting of an amino acid sequence of SEQ ID NO: 1 to C2C12 cells.
FIG. 5 shows the results of confirming, through western blotting, the increased expression level of MyHC, which is a late differentiation marker of myoblasts, after addition of a peptide consisting of an amino acid sequence of SEQ ID NO: 1 to C2C12 cells.
FIG. 6 shows the results of quantitatively evaluating changes in the expression of a late differentiation marker of myoblasts, after addition of a peptide consisting of an amino acid sequence of SEQ ID NO: 1 to C2C12 cells, and confirming the expression level of MyHC.
FIG. 7 shows the results of confirming, through immunofluorescence, the expression pattern of α-actinin, which is a late differentiation marker of myoblasts, after addition of a peptide consisting of an amino acid sequence of SEQ ID NO: 1 to C2C12 cells.
FIG. 8 shows the results of quantitatively evaluating the expression level of α-actinin, after addition of a peptide consisting of an amino acid sequence of SEQ ID NO: 1 to C2C12 cells.
FIG. 9 shows the results of visually confirming changes in TA muscle following treatment with a peptide consisting of an amino acid sequence of SEQ ID NO: 1, in an animal model in which muscle damage was induced by BaCl₂.
FIG. 10 shows the results of quantitatively evaluating changes in muscle mass of TA muscle following treatment with a peptide consisting of an amino acid sequence SEQ ID NO: 1, in an animal model in which muscle damage was induced by BaCl₂, wherein FIG. 10 (a) shows the results of confirming the weight (g) of TA muscle, and FIG. 10 (b) shows the results of confirming the percentage (%) of TA muscle.
FIG. 11 shows the results of confirming, through H&E staining, changes in infiltration areas of immune cells following treatment with a peptide consisting of an amino acid sequence of SEQ ID NO: 1, in muscle tissue derived from an animal model in which muscle damage was induced by BaCl₂.
FIG. 12 shows the results of quantitively evaluating changes in infiltration areas of immune cells following treatment with a peptide consisting of an amino acid sequence of SEQ ID NO: 1, in muscle tissue derived from an animal model in which muscle damage was induced by BaCl₂.
FIG. 13 shows the results of confirming, through sirius red staining, changes in the level of collagen accumulation following treatment with a peptide consisting of an amino acid sequence of SEQ ID NO: 1, in muscle tissue derived from an animal model in which muscle damage was induced by BaCl₂.
FIG. 14 shows the results of quantitively evaluating changes in the level of collagen accumulation following treatment with a peptide consisting of an amino acid sequence of SEQ ID NO: 1, in muscle tissue derived from an animal model in which muscle damage was induced by BaCl₂.
FIG. 15 shows the results of confirming, through western blotting, the increased expression levels of MyHC, which is a protein constituting muscle fibers, and α-actinin, following treatment with a peptide consisting of an amino acid sequence of SEQ ID NO: 1, in muscle tissue derived from an animal model in which muscle damage was induced by BaCl₂.
FIG. 16 shows the results of quantitively evaluating the increased expression levels of MyHC, which is a protein constituting muscle fibers, and α-actinin, following treatment with a peptide consisting of an amino acid sequence of SEQ ID NO: 1, in muscle tissue derived from an animal model in which muscle damage was induced by BaCl₂.
FIG. 17 shows the results of confirming, through western blotting, the decreased expression levels of α-SMA, which is an inflammatory protein, and IL-6, following treatment with a peptide consisting of an amino acid sequence of SEQ ID NO: 1, in muscle tissue derived from an animal model in which muscle damage was induced by BaCl₂.
FIG. 18 shows the results of quantitively evaluating the decreased expression levels of α-SMA, which is an inflammatory protein, and IL-6, following treatment with a peptide consisting of an amino acid sequence of SEQ ID NO: 1, in muscle tissue derived from an animal model in which muscle damage was induced by BaCl₂.

### Best Mode for Carrying out the Invention

### Mode for the Invention

Hereinafter, the present disclosure will be described in detail with reference to Examples below. However, these Examples are for illustrative purposes only, and the scope of the present disclosure is not intended to be limited by these Examples.

### Example 1. Synthesis of peptide

A peptide having an amino acid sequence of SEQ ID NO: 1 shown in Table 1 was synthesized by using an automatic peptide synthesizer (Milligen 9050, by Millipore, USA). Then, C18 reverse-phase high-performance liquid chromatography (HPLC) (by Waters Associates, USA) was performed thereon to purely separate the synthesized peptide. Here, ACQUITY UPLC BEH300 C18 column (2.1 mm X 100 mm, 1.7 µm, Waters Co, USA) was used.

**[Table 1]**

| **SEQ ID NO:** | **N-terminal → C-terminal** |
|---|---|
| 1 | EAEYEHL |

### Example 2. Confirmation of the differentiation-promoting effects of myoblasts at early differentiation stage

In this example, by evaluating changes in the expression of an early differentiation marker protein of C2C12 cells, which are mouse myoblasts, it was to confirm the effects of the peptide according to an example on the differentiation of myoblasts into muscle cells/muscle fibers at an early differentiation stage.

### 2-1. Evaluation through protein expression analysis

The C2C12 cells were cultured in a DMEM supplemented with 1 % P/S and 2 % BCS. When the cell confluency reached 70-80 %, the cells were seeded into 6-well culture plates. Afterwards, when the cell confluency in the 6-well culture plate reached 100 %, the culture medium was replaced with a differentiation medium (supplemented with 1 % P/S and 2 % horse serum), and 5, 50, or 100 ug/ml of the peptide of SEQ ID NO: 1 was added thereto. Afterwards, the differentiation medium and the same amount of the peptide of SEQ ID NO: 1 were replaced and added, respectively, once every two days. After three days from the first day of medium replacement, the culture was lysed by adding a dissolution buffer thereto, and proteins were obtained by centrifugation at 4°C and 12,000 rpm for 30 minutes. These proteins were then quantified by using a BCA kit. SDS-PAGE was performed on samples containing these proteins to separate the respective proteins which were then electrotransferred onto a membrane. The membrane to which the proteins were attached was blocked by treating with 5 % skim milk, and primary antibodies were allowed for a reaction overnight at 4 °C. After washing with PBS-T, secondary antibodies were allowed for a reaction at room temperature for 1 hour. After washing with PBS-T again, the proteins were detected by using Gel Doc (Bio-Rad, Hercules, CA, USA) and Western detection reagents (Elpis Biotech, Daejeon, Korea). Here, anti-MyoG (santa cruz, USA), anti-Myf5 (Abcam, UK), and anti-GAPDH (santa cruz, USA) were used as the primary antibodies, a non-treated group (Non) was used as a control group, and a group (CM) treated with a differentiation medium only was used as a comparison group.

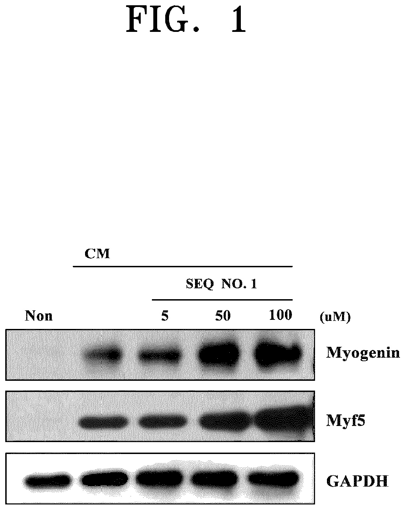

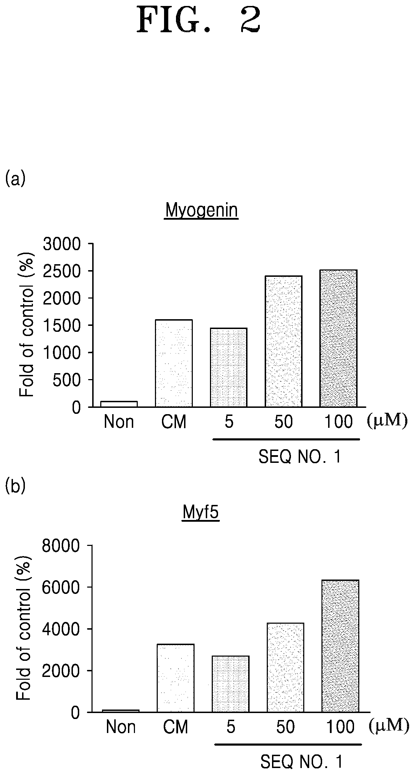

As a result, as shown in FIGS. 1 and 2, it was confirmed that the expression of myogenin (MyoG) and Myf5, which are early differentiation markers of myoblasts, was increased by the treatment with the peptide of SEQ ID NO: 1.

### 2-2. Evaluation by immunofluorescence method

C2C12 cells were cultured in a DMEM supplemented with 1 % P/S and 2 % BCS. When the cell confluency reached 70-80 %, the cells were seeded into a 6-well culture plate. Afterwards, when the cell confluency in the 6-well culture plate reached 100 %, the culture medium was replaced with a differentiation medium (supplemented with 1 % P/S and 2 % horse serum), and 100 ug/ml of the peptide of SEQ ID NO: 1 was added thereto. Afterwards, the differentiation medium and 100 ug/ml of the peptide were replaced and added, respectively, once every two days. After three days from the first day of medium replacement, the cultured cells were fixed with 4 % paraformaldehyde (PFA) at room temperature for 10 minutes and permeabilized with 0.1 % triton X-100 for 5 minutes. Afterwards, the fixed cells were mixed with PBS, 10 % FBS, and 0.1 % triton X-100 for blocking at room temperature for 1 hour, and then treated with primary antibodies (MyoG, Santa Cruz, USA). The resulting cells were incubated for 1 hour at room temperature. Afterwards, the cells were washed, and incubated again with secondary antibodies (goat anti-mouse IgG-TR, Santa Cruz, USA) at room temperature for 30 minutes. Afterwards, the cell nuclei were stained with DAPI (Santa Cruz, USA) and observed under a LEICA fluorescence microscope (TCS SP8, LEICA, Germany).

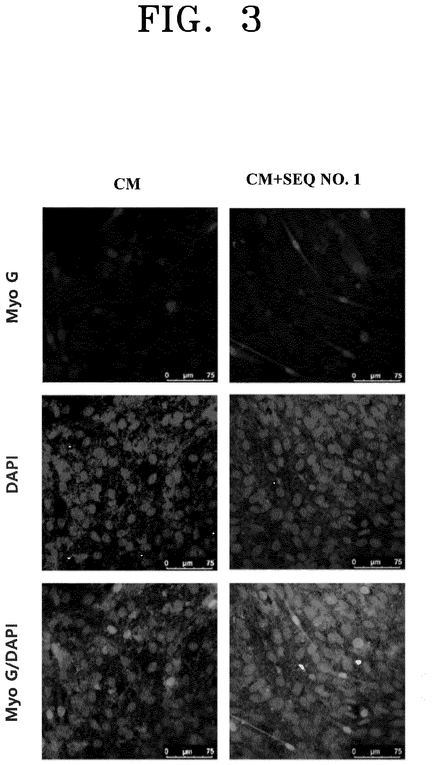

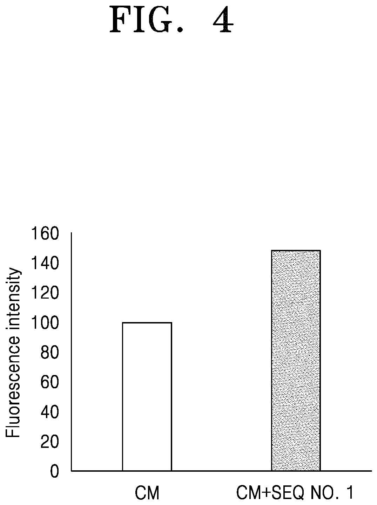

As a result, as shown in FIGS. 3 and 4, it was confirmed that, by the treatment with the peptide of SEQ ID NO: 1, not only was the expression of myogenin (MyoG), which is an early differentiation marker of myoblasts, increased, but also the expression in the nuclear region of myoblasts was increased.

To sum up the experimental results, it was confirmed that the peptide according to an embodiment contributes to promoting the early differentiation of myoblasts into muscle cells.

### Example 3. Confirmation of differentiation-promoting effects of myoblasts at late differentiation stage

In this example, by evaluating changes in the expression of an early differentiation marker protein of C2C12 cells, which are mouse myoblasts, it was to confirm the effects of the peptide according to an example on the differentiation of myoblasts into muscle cells/muscle fibers at a late differentiation stage.

### 3-1. Evaluation through protein expression analysis

The same method as Example 2-1, except for the time of lysis buffer addition (after 7 days from the first day of medium replacement), was performed to determine the expression level of a myosin heavy chain (MyHC) protein, which is a late differentiation marker of myoblasts. Here, anti-MyHC (santa cruz, USA) and anti-Myf5 (Abcam, UK), and anti-GAPDH (santa cruz, USA) were used as the primary antibodies, a non-treated group (Non) was used as a control group, and a group (CM) treated with a differentiation medium only was used as a comparison group.

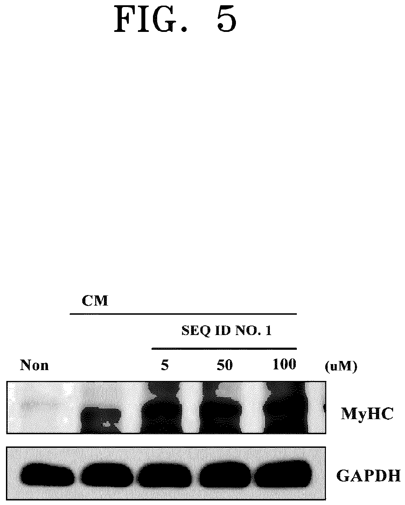

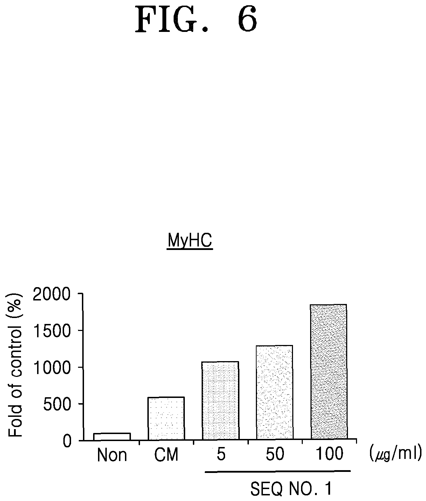

As a result, as shown in FIGS. 5 and 6, it was confirmed that the expression of MyHC, which is a late differentiation marker of myoblasts, was increased by the treatment with the peptide of SEQ ID NO: 1.

### 3-2. Evaluation by immunofluorescence

The same method as Example 3-1, except for the time of fixing (after 7 days from the first day of medium replacement), was performed to determine the expression level of MyHC protein, which is a late differentiation marker of myoblasts. Here, anti-α-actinin (Santa Cruz, USA) was used as the primary antibody, goat anti-mouse IgG-TR (Santa Cruz, USA) was used as the secondary antibody, and a group (CM) treated with a differentiation medium only was used as a control group.

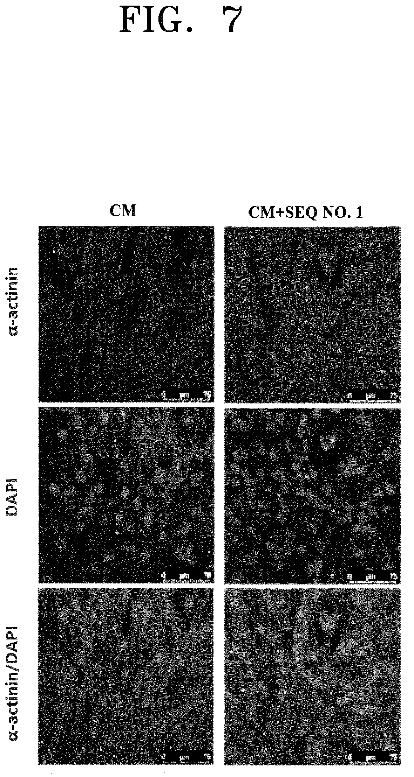

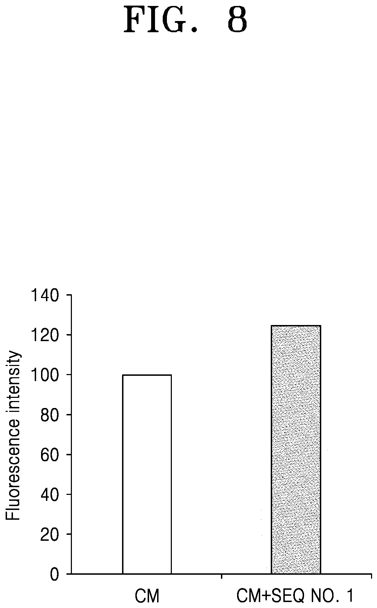

As a result, as shown in FIGS. 7 and 8, it was confirmed that the expression of α-actinin, which is a late differentiation marker of myoblasts, was increased by the treatment with the peptide of SEQ ID NO: 1.

To sum up the experimental results, it was confirmed that the peptide according to an embodiment contributes to promoting the late differentiation of myoblasts into muscle cells.

### Example 4. Confirmation of muscle loss prevention effects by using animal model

In this example, an animal model of muscle damage induced by BaCl₂ was used to determine the effect of the peptide according to an embodiment on the muscle loss prevention, by evaluating changes in muscle mass, areas of infiltration of immune cells in muscle tissue, levels of accumulated collagen, and expression levels of proteins constituting muscle fibers and inflammatory proteins.

### 4-1. Evaluation of muscle mass

50 µl of a 1.2 % (w/v) BaCl₂ aqueous solution was injected into the tibialis anterior (TA) muscle of a 8-week-old male C57BL/6 mouse to induce muscle damage for 1 day. Afterwards, 20 mg of the peptide of SEQ ID NO: 1 was administered orally at a concentration of 0.1 mg/µl at 1-day intervals for a total of six times to the mouse with induced muscle damage. For a positive control group, 0.5 ug of IGF-I was injected intramuscularly at a concentration of 0.01 ug/µl. On Day 7 after inducing the muscle damage, the mouse was sacrificed, and the changes in the TA muscle were visually observed and the mouse was weighed. A non-treated group (Non) was used as a control group, a group (N.C) in which only muscle damage was induced by BaCl₂ was used as a negative control group, and a group (P.C) in which muscle damage was induced by BaCl₂ and then treated with IGF-I was used as a positive control group.

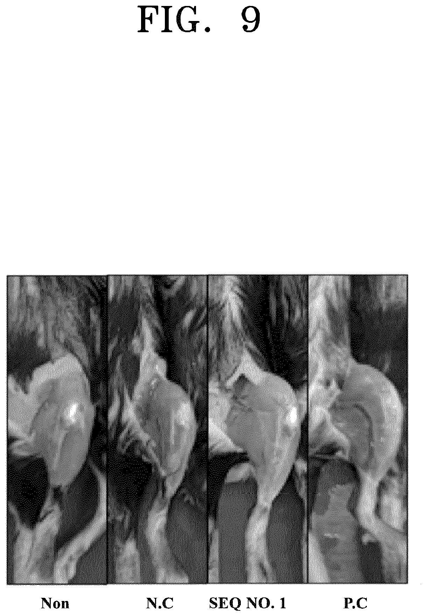

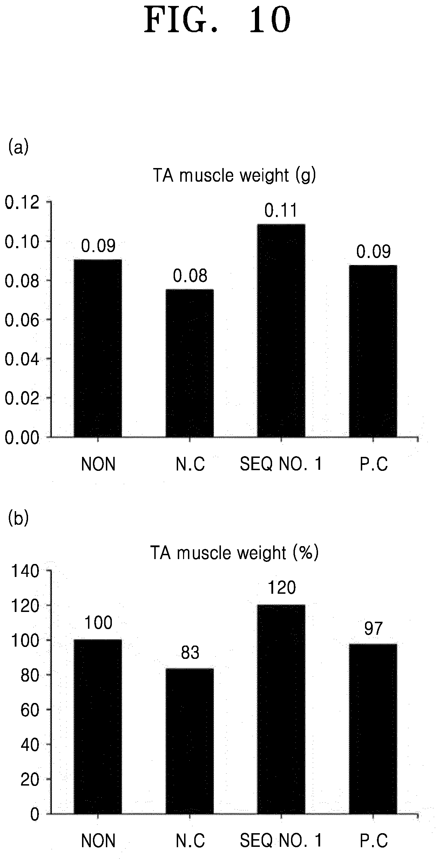

As a result, as shown in FIGS. 9 and 10, it was confirmed that, by the treatment with the peptide of SEQ ID NO: 1, the muscle mass of the TA muscle of the mouse in which muscle damage was induced by BaCl₂ was increased, indicating that it can promote regeneration of damaged muscles.

### 4-2. Evaluation by muscle tissue staining

Muscle tissue obtained from the mouse with induced muscle damage of Example 4-1 was washed with PBS at room temperature and then fixed with 4 % paraformaldehyde (PFA). Afterwards, the fixed muscle tissue was washed three times with PBS and dehydrated by using a gradient ethanol series (from 70 % to 100 %). A sample of the muscle tissue was sectioned into 4 µm sections. A tissue slide containing the sectioned specimens were dewaxed and hydrated through a gradient ethanol series, and sequentially stained in a hematoxylin solution for 1 minute and in an eosin solution for 10 seconds. After the staining, the tissue slide was sequentially immersed in 90 % ethyl alcohol and 100 % ethyl alcohol, and finally immersed twice in xylene for 5 minutes each, and then mounted (H&E staining).

In addition, the tissue slide containing the sectioned specimens was dewaxed and hydrated as described above, stained in a picro sirius red solution for 60 minutes, immersed twice in a 1 % acetic acid solution for 2 minutes each, and washed with D.W. Afterwards, the tissue slide was immersed in ethyl alcohol, and finally immersed twice in xylene for 5 minutes each, and then mounted (Sirius red staining).

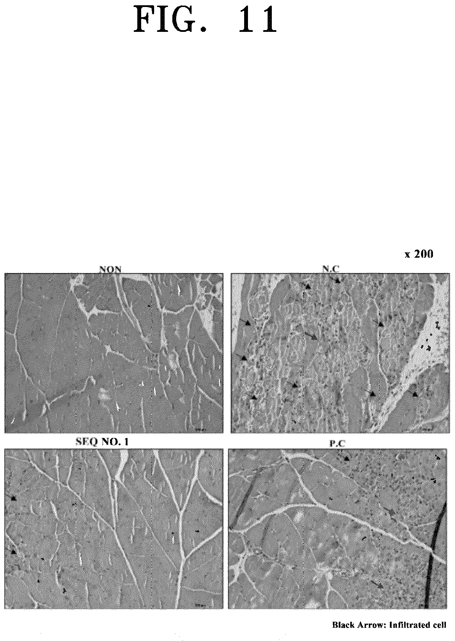

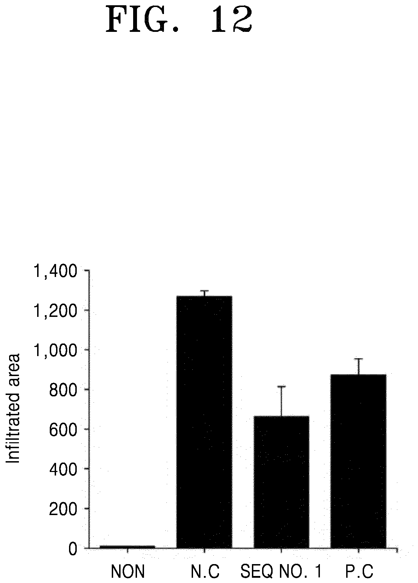

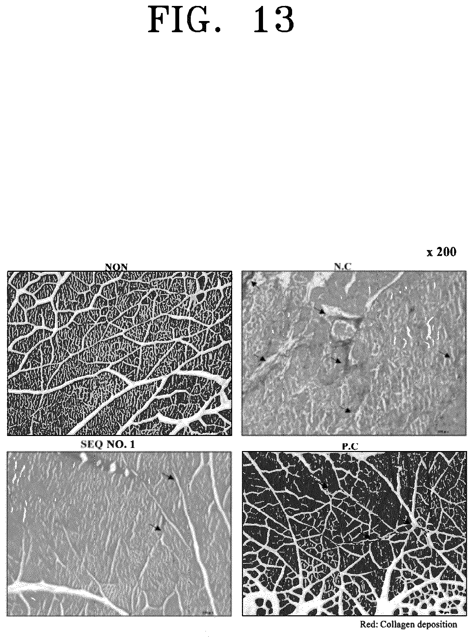

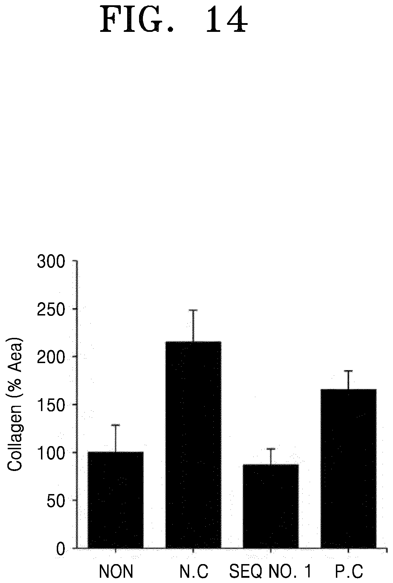

As a result, as shown in FIGS. 11 and 12, it was confirmed that, by the treatment of the peptide of SEQ ID NO: 1, the area of infiltration of immune cells in the tissue in which the muscle damage was induced by BaCl₂ was reduced. In addition, as shown in FIGS. 13 and 14, it was confirmed that, by the treatment of the peptide of SEQ ID NO: 1, the accumulation of collagen in the tissue in which the muscle damage was induced by BaCl₂ was reduced.

### 4-3. Evaluation through analysis of protein expression in muscle tissue

The muscle tissue obtained from the mouse with the induced muscle damage of Example 4-1 was lysed by using homogenizer, and the lysate was dissolved by adding a dissolution buffer thereto. The resulting product was centrifuged at 4 °C and 12,000 rpm for 30 minutes to obtain proteins which were then quantified by using a BCA kit. SDS-PAGE was performed on samples containing these proteins to separate the respective proteins which were then electrotransferred onto a membrane. The membrane to which the proteins were attached was blocked by treating with 5 % skim milk, and primary antibodies were allowed for a reaction overnight at 4 °C. After washing with PBS-T, secondary antibodies were allowed for a reaction at room temperature for 1 hour. After washing with PBS-T again, the proteins were detected by using Gel Doc (Bio-Rad, Hercules, CA, USA) and Western detection reagents (Elpis Biotech, Daejeon, Korea). Here, anti-MyHC (Santa Cruz, USA), anti-α-actinin (Santa Cruz, USA), anti-α-SMA (Abcam, UK), anti-IL-6 (Santa Cruz, USA), and anti-α-tubulin (Santa Cruz, USA) were used as the primary antibodies.

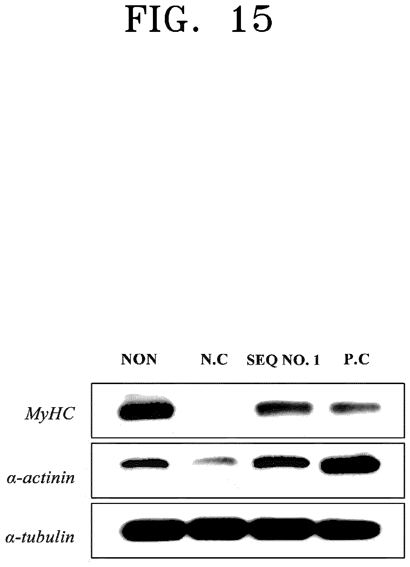

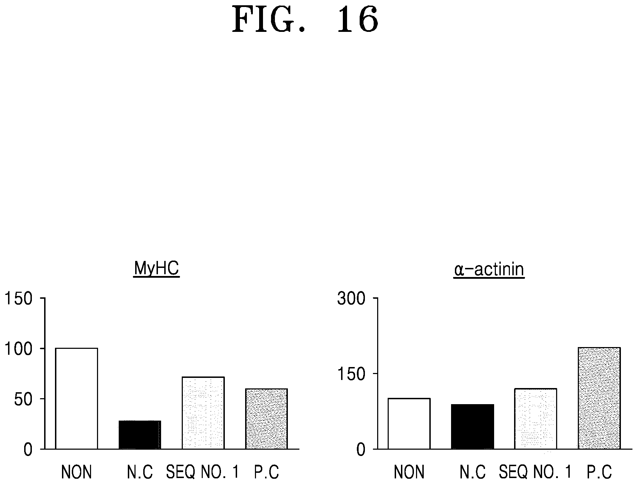

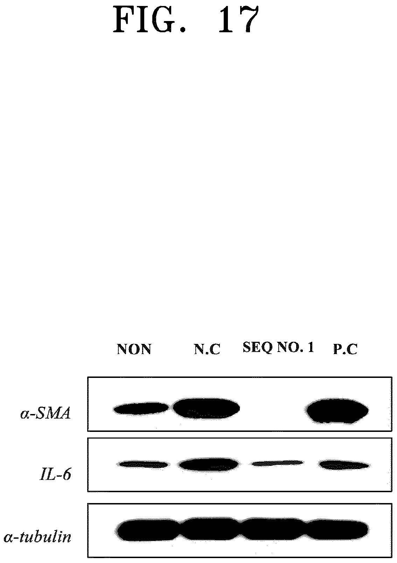

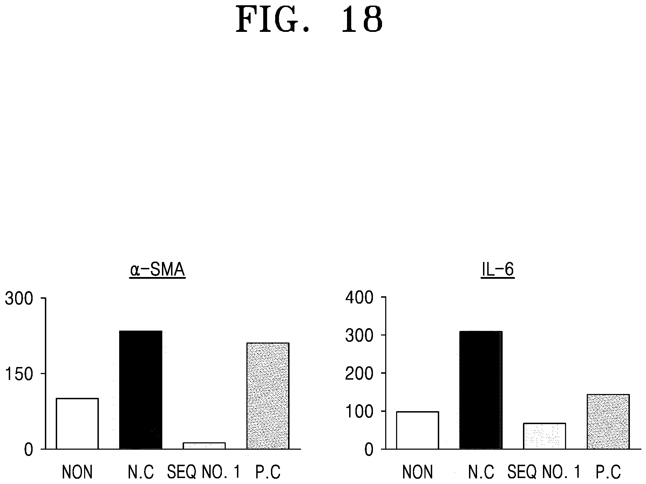

As a result, as shown in FIGS. 15 and 16, it was confirmed that, by the treatment of the peptide of SEQ ID NO: 1, the expression of MyHC which is a protein constituting muscle fibers and the expression of α-actinin were increased in the tissue in which muscle damage was induced by BaCl₂. In addition, as shown in FIGS. 17 and 18, it was confirmed that, by the treatment of the peptide of SEQ ID NO: 1, the expression of α-SMA which is an inflammatory protein and the expression of IL-6 were decreased in the tissue in which muscle damage was induced by BaCl₂.

To sum up with the experimental results, it was confirmed that the peptide according to an embodiment contributes not only to regenerating or recovering damaged muscles, but also to suppressing progression of muscle damage such as inflammation causing muscle damage.

### Formulation Example 1. Preparation of peptide nanosomes

50 mg of the peptide of Example 1 was dissolved in 500 ml of distilled water with sufficient stirring. The mixed solution was mixed with 5 g of lecithin, 0.3 ml of sodium oleate, 50 ml of ethanol, and a small amount of oil, and the total volume was adjusted to 1 L with distilled water. The resulting solution was then emulsified by using a microfluidizer at high pressure to prepare peptide nanosomes with a size of about 100 nm.

### Formulation Example 2. Pharmaceutical formulations

### 2-1. Preparation of powder formulation

A powder formulation was prepared by mixing the following ingredients and filling an airtight bag with the mixed ingredients.
20 mg of peptide disclosed herein
100 mg of lactose
10 mg of talc

### 2-2. Preparation of tablet formulation

A tablet formulation was prepared by mixing the following ingredients and forming the mixed ingredients into tablets according to a conventional tableting process.
10 mg of peptide disclosed herein
100 mg of corn starch
100 mg of lactose
2 mg of magnesium stearate

### 2-3. Preparation of capsule formulation

A capsule formulation was prepared by mixing the following ingredients and filling a gelatin capsule with the mixed ingredients according to a conventional capsule formation method.
10 mg of peptide disclosed herein
3 mg of crystalline cellulose
14.8 mg of lactose
0.2 mg of magnesium stearate

### 2-4. Preparation of injection formulation

According to a conventional method for preparing an injection formulation, an injection was prepared based on the following ingredient contents per 1 ampoule (2 ml).
10 mg of peptide disclosed herein
180 mg of mannitol
2,974 mg of sterile distilled water for injection
26 mg of Na₂HPO₄·2H₂O

### 2-5. Preparation of liquid formulation

According to a conventional method for preparing a liquid formulation, each component was added to purified water and dissolved therein, and then mixed with the following components. Purified water was added thereto so that the total volume was adjusted to 100 ml, and the resulting solution filled a brown bottle and sterilized, thereby preparing a liquid formulation.
10 mg of peptide disclosed herein
10 g of isomerized glucose
5 g of mannitol
Appropriate amount of purified water

The foregoing descriptions are only for illustrating the disclosure, and it will be apparent to a person having ordinary skill in the art to which the present invention pertains that the embodiments disclosed herein can be easily modified into other specific forms without changing the technical spirit or essential features. Therefore, it should be understood that Examples described herein are illustrative in all respects and are not limited.

## Claims

1. A peptide consisting of an amino acid sequence of SEQ ID NO: 1.

2. The peptide of claim 1, wherein an N-terminus of the peptide is bound to any one protecting group selected from the group consisting of an acetyl group, a fluoreonylmethoxycarbonyl group, a formyl group, a palmitoyl group, a myristyl group, a stearyl group, a butoxycarbonyl group, an allyloxycarbonyl group, and polyethylene glycol (PEG).

3. The peptide of claim 1, wherein a C-terminus of the peptide is bound to any one protecting group selected from the group consisting of an amino group (-NH₂), a tertiary alkyl group, and an azide group (-NHNH₂).

4. The peptide of claim 1, wherein the peptide exhibits at least one of the following characteristics:
(a) enhanced expression of myogenin and myogenic factor 5;
(b) enhanced expression of myosin heavy chain and α-actinin; and
(c) reduced expression of α-smooth muscle actin and interleukin-6.

5. A pharmaceutical composition for preventing or treating a muscle disease, comprising the peptide of any one of claims 1 to 4 as an active ingredient.

6. The pharmaceutical composition of claim 5, wherein the muscle disease is caused by decreased muscle function, muscle wasting, or muscle degeneration.

7. The pharmaceutical composition of claim 6, wherein the muscle disease is any one of sarcopenia, atony, muscular dystrophy, muscular atrophy, muscle degeneration, myotonia, amyotrophic lateral sclerosis, myasthenia, and cachexia.

8. A food composition for preventing or ameliorating a muscle disease, comprising the peptide of any one of claims 1 to 4 as an active ingredient.

9. The food composition of claim 8, wherein the muscle disease is caused by decreased muscle function, muscle wasting, or muscle degeneration.

10. The food composition of claim 8, additionally comprising any one ingredient selected from vitamin C, vitamin B1, vitamin B2, vitamin B6, vitamin H, vitamin PP, pro-vitamin B5, vitamin A, vitamin D, vitamin E, vitamin K1 or carotene, or mixtures thereof.
